# EUROPEAN PATENT APPLICATION

(11) **EP 4 032 541 A1**
(43) Date of publication of application: **27.07.2022**
(21) Application number: 20866272.6
(22) Date of filing: 22.04.2020
(51) Int. Cl.: A61K 38/38, A61K 47/10, A61K 47/12, A61P 7/08

(54) **PREPARATION CONTAINING HUMAN ALBUMIN AND PREPARATION METHOD THEREFOR**

(30) Priority: 17.09.2019 CN 201910874343
(71) Applicant: Tonghua Anrate Biopharmaceutical Co., Ltd, Tonghua, Jilin 134000 (CN)
(72) Inventor: XIANG, Wei, Tonghua, Jilin 134000 (CN); YUE, Zhilei, Tonghua, Jilin 134000 (CN)
(74) Representative: Miao, Tianhao
(86) International application number: PCT/CN2020/086057
(87) International publication number: WO 2021/051807

(57) **Abstract**

Disclosed in the present invention is a preparation method of a human albumin-containing medical product, comprising: improving the stability of human albumin in the medical product by controlling the content of long-chain fatty acids in the medical product and adding poloxamer to the medical product.

## Description

### FIELD OF TECHNOLOGY

The present invention relates to the field of biomedicines, and particularly to a human albumin-containing medical product and preparation method thereof. In the present invention, the term "human albumin" comprises human serum albumin and recombinant human albumin. The recombinant human albumin is also called recombinant human serum albumin or recombinant human albumin mutant.

### BACKGROUND

The main pharmacological effects of human albumin include regulating the dynamic equilibrium of water between tissues and blood vessels, maintaining a normal and constant plasma volume, and transport certain ions and compounds by reversibly binding to them due to a high affinity for these substances. Moreover, human albumin provides a large amount of amino acid reserve for the body. The above-mentioned effects of human albumin allow it to be used in various clinical disciplines for a variety of therapeutic purposes. Human albumin is mainly used clinically to regulate plasma colloid osmotic pressure, expand blood volume, treat traumatic and hemorrhagic shock, severe burns and hypoproteinemia, and is widely used for common diseases such as stroke, cirrhosis and hepatic ascites, and kidney diseases. Besides of the direct application in the field of clinical therapeutics, albumin has been extremely widely used in multiple aspects, such as culture media used for vaccine production, pharmaceutical excipients, diagnostic reagents, novel long-acting drug product for the treatment of tumors, cosmetics, and laboratory bioreagents.

Human albumin is a single-chain non-glycosylated protein with a heart-shaped structure and a molecular weight of 66,438 Da, composed of 585 amino acids, 17 pairs of disulfide bridges and one free sulfydryl group. The half-life thereof in human is between 19 and 21 days. The heart-shaped structure of human albumin is composed of three main domains that are loosely joined together through the van der Waals force and six subdomains that are wrapped by 17 disulfide bridges, as revealed by its crystal structure. The disulfide bridges impart rigidity to the helical, globular structure but provide enough flexibility to allow the protein to undergo conformational changes in response to variations in the surrounding medium.

Human albumin is conventionally produced by fractionation, extraction and purification from human serum, collectively known as human serum albumin. Human serum albumin has been discovered and used for decades with well-developed and complete production and formulation processes (Cohn method - ultrafiltration -/ or purification - pasteurization - formulation (sodium caprylate and/or N-acetyltryptophan)). The formulation thereof has become a general standard for national pharmacopoeias worldwide. Human serum-derived albumin features good stability. According to pharmacopoeial requirements, the final product shall be kept in a water bath of 57°C±0.5°C for 50 hours, and no visual changes shall appear except slight change in color. All final products of serum-derived albumin shall be kept at 20-25°C for at least 4 weeks or at 30-32°C for at least 14 days. Relevant tests shall be carried out on the containers where turbidity or smoky precipitate is shown. Even though such strict thermostability test is performed on human serum albumin, phlebitis related to micro-aggregates and protein fibrillar aggregates still occur clinically. Clinical studies have demonstrated a 20% incidence of phlebitis in patients when ordinary infusion sets with 15 µm filters were used for IV infusion of human serum albumin, but only 2.5% when precision infusion sets with 5 µm filters were used. Hence it was demonstrated that after sterilization and particle removal by 0.1-0.22 µm filtration during the production process of human serum albumin, new micro-aggregates and micro-fibrillar proteins still appeared in the medical product during shipping and storage, leading to a large proportion of albumin-infusion induced phlebitis in patients (Xiao-Ping Xue et al., Journal of Clinical Nursing, 2010 Oct, 9(5): 69-70). Therefore, requirements for precision infusion sets are specified in the specifications of human serum albumin by some manufacturers. Even so, the above-mentioned partially unstable albumin is 30-100 nm in diameter and hence cannot be effectively removed by common sterile membranes (0.1-0.22 µm). Aggregates of this diameter with sizes between polymers and visible particles, are a major cause of adverse reactions such as immunogenicity.

Another source of human albumin is recombinant human albumin expressed by hosts capable of specifically expressing human albumin, such as Pichia pastoris or Saccharomyces cerevisiae, the single-celled fungus microorganisms cultivated by the method of genetic recombination, or recombinant human albumin expressed by transgenic rice. However, the highly-purified final product of recombinant human albumin, complying with the same formulation requirements specified in pharmacopoeias, is far less stable than human serum-derived albumin. In view of this drawback, a Chinese patent application (application number CN201610017874.7) disclosed a method to prevent the formation of recombinant human albumin aggregates by adding Tween 80 or Tween 20 on the basis of pharmacopoeia standard formulation.

S. Baldursdottir et al. disclosed the stabilizing effect of excipient polysorbate 80 (Tween 80) in recombinant human albumin solutions. It was observed that the excipients caprylate and polysorbate 80 used in the Recombumin Alpha^{®} formulation both increased the stability of the protein. Caprylate seems to be efficient in protecting the protein at higher temperatures, whereas polysorbate (Tween) protects against self-association at lower temperatures. Additionally, a synergistic effect of caprylate and polysorbate 80 was observed in a non-linear fashion. (S. Baldursdottir, M. Tauhaybeche, J. Pajander, J.T. Bukrinski, L. Jorgensen, Screening of formulation parameters for stabilizing recombinant human serum albumin (rHSA) in liquid formulations, Journal of Drug Delivery Science and Technology (2016), doi: 10.1016/j.jddst.2016.05.001.)

The negative effect of polysorbate lies in an increased possibility of protein oxidation catalysed by residual alkyl oxides in the non-ionic surfactant. For example, recombinant human ciliary neurotrophic factor (CNTF) can be dimerized by the alkyl peroxide in polysorbate 80. The level of alkyl peroxides in polysorbate 80 correlates with the degree of oxidation of recombinant human granulocyte-colony stimulating factor (r hG-CSF), and peroxide-mediated oxidation seems to be more severe than that mediated by atmospheric oxygen present in the vial headspace (Knepp VM, Whatley JL, Muchnik A, Calderwood TS. 1996. Identification of antioxidants for prevention of peroxide-mediated oxidation of recombinant human ciliary neurotrophic factor and recombinant human nerve growth factor. PDA J Pharm Sci Technol 50(3):163-171; Herman AC, Boone TC, Lu HS. 1996. Characterization, preparation, and stability of Neupogen (Filgrastim), a recombinant human granulocyte-colony stimulating factor. Pharm. Biotechnol. 9:303-328).

Human albumin comprises methionine and a free sulfhydryl group for protection of cells and organs against damages due to peroxidation by free radicals. Therefore, the quality and storage conditions of polysorbate need to be well controlled, and the correlation of their amount in proteins needs to be kept at a minimum level (AGGREGATION OF THERAPEUTIC PROTEINS, P341, Edited by Wei Wang, Christopher J. Roberts, WILEY 2010), otherwise human albumin will be partially oxidized, losing its proper redox physiological activity; meanwhile, the oxidized human albumin will further facilitate the unfolding of albumin, accelerating the aggregation and fibrillation of human albumin.

One of the very important physiological functions of human albumin in circulation is to transport drugs, fatty acids, vitamins, cytochromes and metal ions based on its ligand binding properties.

Fatty acids, as ligands of human albumin, play a decisive role in the structure and thermostability of human albumin. The addition of sodium caprylate as a ligand stabilizer to human serum albumin can effectively increase the values of enthalpy and Tm (melting temperature), thus enhancing the thermostability.

Brian et al. disclosed the use of differential scanning calorimetry (DSC) to determine the unfolding properties of commercial products of human albumin prepared from pooled human blood, transgenic yeast, and transgenic rice. After the addition of oleic acid, the initial melting temperatures (Tm1) for the unfolding transitions of the human albumin products increased diversely from 62°C to 75°C. This study demonstrated the importance of fatty acid ligands in the thermostability of human albumin, however, aggregation and fibrillation due to exposure of the hydrophobic region of the protein was not investigated, and DSC could not characterize the aggregation and fibrillation at low and ambient temperatures and under oscillation conditions during shipping. (Brian E. Lang and Kenneth D. Cole, Biotechnol. Prog., 2014, Unfolding Properties of Recombinant Human Serum Albumin Products Are Due To Bioprocessing Steps, DOI 10. 1002/btpr. 1996).

In addition to fatty acids, there are additional ligands such as bilirubin, hormones, lipid-soluble drugs, and heme that are tightly bound to human serum-derived albumin, increasing the conformational stability of albumin. Defatted human serum-derived albumin shows significantly decreased stability, which is in complete agreement with highly purified recombinant human albumin.

Surfactants such as Tween as stabilizers shall be added as little as possible to avoid their toxic effects and, more importantly, to reduce the interference of Tween and other surfactants with the physiological effects of human albumin ligands. Otherwise safer surfactants at lower doses shall be searched for.

Poloxamer is the generic name of polyoxyethylene-polyoxypropylene copolymers. Poloxamers, produced by BASF in Germany and known by the trade name Pluronic, are non-ionic surfactants. Poloxamers are the only synthetic emulsifiers currently used in intravenous emulsions, of which poloxamer 188 has the best emulsifying performance and safety as an O/W emulsifier with a commonly used concentration of 0.3% (Wang Meng et al., Pharmaceutical and Clinical Research. 2007, 15(1): 10-13).

As a non-ionic surfactant, poloxamer 188 can enhance the solubility of insoluble drugs by forming micelles with a critical micelle concentration (CMC) of about 0.6%. Poloxamer 188 can inhibit thrombosis, affect blood rheological activity, cell membrane sealing, phagocyteic cell activation (stimulated phagocytosis and production of super negative ions) and degranulation of neutrophils, and prevent skeletal muscle necrosis in adults. In addition, poloxamer 188 can be used to treat brain injury. Studies have shown that poloxamer 188 can reduce inflammation and tissue damage in rats due to experimental brain injury. Moreover, evidence in other experimental models have demonstrated that it counters tissue damage. The protective mechanism thereof may relate to the effects of the surfactant on oxidative stress and inflammatory reaction. Moreover, poloxamer 188 can enhance cell viability. Studies have demonstrated that a single intravenous dose of poloxamer 188 is effective to restore damaged tissues with intact circulation. Poloxamer 188 is capable of stabilizing defects in cell membranes arising from a variety of causes. Studies in rats have shown that, a single dose of poloxamer 188 counters early neuronal damage after hemorrhage, but is ineffective on neuronal damage caused by long-term hemorrhage. However, daily administration of poloxamer 188 may generate long-term and effective neuronal protection.

Chinese patent application under application number CN200480003079.8 disclosed the use of poloxamer as a surfactant to stabilize hematopoietic factors, parathyroid hormones or antibodies. There was no ligand effect of fatty acids on the proteins in this patent, hence a larger dose of poloxamer was added to stabilize the proteins and to avoid oxidative injury by Tween. Since hematopoietic factors, parathyroid hormones or antibodies are small volume injections (ug-mg/vial), the total poloxamer is not significant even for addition of larger concentrations of poloxamer. However, human albumin is a large volume injection with an injection dosage of 5-12.5 g/vial, hence the total Tween or total poloxamer is larger even if a small concentration of Tween or poloxamer is added.

Therefore, those of skill in the art are dedicated to the study of a human albumin-containing medical product or preparation method thereof to inhibit the formation of aggregates and fibrillar micro-particles of human albumin and to increase the safety and tolerability of human albumin in clinical use, while avoiding toxicity of Tween and oxidative denaturation of proteins caused by Tween oxides in published formulations.

### SUMMARY

The technical issue to be solved by the present invention is to improve the stability of human albumin preparations.

To address the to-be-solved technical issue, the present invention provides a preparation method of a human albumin-containing medical product, the method comprising: improving the stability of human albumin in the medical product by controlling the content of long-chain fatty acids in the medical product and by adding poloxamer to the medical product.

Preferably, the method further comprises: improving the stability of human albumin in the medical product by reducing aggregation and fibrillation of human albumin.

Preferably, the method further comprises: improving the stability of human albumin in the medical product so that no particle of over 30 nm in diameter is formed from aggregation and/or fibrillation of human albumin after the preparation of the medical product.

Preferably, the method further comprises: improving the stability of human albumin in the medical product so that no particle of over 100 nm in diameter is formed from aggregation and/or fibrillation of human albumin after the preparation of the preparation.

Preferably, the method further comprises: improving the stability of human albumin in the medical product so that no particle of about 200 nm in diameter is formed from aggregation and/or fibrillation of human albumin after the preparation of the preparation.

Preferably, the method further comprises: improving the stability of human albumin in the medical product so that the stability meets requirements in local pharmacopoeias.

Preferably, the method further comprises: improving the stability of human albumin in the medical product so that the stability meets the following conditions: no turbidity or visible particles are formed during incubation at 57°C for 50 hours, at 30°C for 14 days and at 20°C for 28 days.

Preferably, the human albumin is human serum albumin, and the method comprises: improving the stability of human albumin in the medical product by adding poloxamer.

Preferably, the human albumin is recombinant human albumin, and the method comprises: improving the stability of human albumin in the medical product by adding long-chain fatty acids and poloxamer.

Preferably, the long-chain fatty acid is added in the form of a salt.

Preferably, the long-chain fatty acid is added in the form of a sodium salt.

Preferably, the method further comprises: after the preparation of the medical product, the molar ratio of the long-chain fatty acids and human albumin is 0.2-3.0.

Preferably, the method further comprises: after the preparation of the medical product, the molar ratio of the long-chain fatty acids and human albumin is 0.3-2.0.

Preferably, the method further comprises: after the preparation of the medical product, the content of the poloxamer is 10-500 µg/ g of human albumin.

Preferably, the poloxamer is one or more selected from poloxamer 124, poloxamer 188, poloxamer 237, poloxamer 338 and poloxamer 407.

Preferably, the poloxamer is poloxamer 188.

Preferably, the long-chain fatty acid is one or more selected from myristic acid (C14:0), palmitic acid (C16:0), stearic acid (C18:0), oleic acid (C18:1), linoleic acid (C18:2), linolenic acid (C18:3) and arachidonic acid (C20:4).

Preferably, the long-chain fatty acid is soya fatty acid or fully hydrogenated soya fatty acid.

Preferably, the method further comprises: adding ingredients and contents required by local pharmacopoeias.

Preferably, the ingredients required by local pharmacopoeias comprise sodium caprylate or/and N-acetyltryptophan.

Further provided in the present invention is a human albumin-containing medical product prepared according to the method provided in the present invention.

Further provided in the present invention is a human albumin-containing medical product, which contains no particle of over 30 nm in diameter due to aggregation and/or fibrillation of human albumin.

Further provided in the present invention is a human albumin-containing medical product, which contains no particle of over 100 nm in diameter due to aggregation and/or fibrillation of human albumin.

Further provided in the present invention is a human albumin-containing medical product, which contains no particle of about 200 nm in diameter due to aggregation and/or fibrillation of human albumin.

Further provided in the present invention is a human albumin-containing medical product, the method further comprising: the stability of human albumin in the medical product meets the following conditions: no turbidity or visible particles are formed during incubation at 57°C for 50 hours, at 30°C for 14 days and at 20°C for 28 days.

Further provided in the present invention is a human albumin-containing medical product, which comprises poloxamer and long-chain fatty acids.

Preferably, the molar ratio of the long-chain fatty acids and human albumin in the medical product is 0.2-3.0.

Preferably, the molar ratio of the long-chain fatty acids and human albumin in the medical product is 0.3-2.0.

Preferably, the method further comprises: the content of the poloxamer in the medical product is 10-500 µg/ g of human albumin.

Preferably, the poloxamer is one or more selected from poloxamer 124, poloxamer 188, poloxamer 237, poloxamer 338 and poloxamer 407.

Preferably, the poloxamer is poloxamer 188.

Preferably, the long-chain fatty acid is one or more selected from myristic acid (C14:0), palmitic acid (C16:0), stearic acid (C18:0), oleic acid (C18:1), linoleic acid (C18:2), linolenic acid (C18:3) and arachidonic acid (C20:4).

Preferably, the long-chain fatty acid is soya fatty acid or fully hydrogenated soya fatty acid.

Preferably, the medical product further comprises ingredients and contents required by local pharmacopoeias.

Preferably, the ingredients required by local pharmacopoeias comprise sodium caprylate or/and N-acetyltryptophan.

The present invention is to improve the stability of human album on the basis of reduced heat denaturation via using fatty acid ligands, and stability is improved by reducing aggregation and fibrillation at low, ambient and high temperatures or during shipping oscillation, on the basis of using poloxamer as hydrophobic patches shielding hydrophobic interactions and interfacial hydrophobic interactions. Such aggregates and fibrillar particles are of 30-300 nm in diameter, mainly between 100-200 nm, which can neither be detected by HPLC-SEC test, nor effectively filtered by 0.1-0.2 µm membrane systems, and more importantly, such aggregates are continuously formed in unstable albumin solution systems (even if the aggregates and fibrillar particles have been completely removed) during storage and shipping, and keep growing into visible particles with a diameter of micron and sub-millimeter level.

In order to achieve inhibition of aggregation and fibrillation of human albumin under various conditions, the inventors of the present application have conducted extremely detailed studies to dissect human plasma-derived albumin. It was found that 0.3 to 0.8 mol of long-chain fatty acids are contained in each mol of human plasma-derived albumin. In contrast, 0.5 to 2.5 mol of long-chain fatty acids are contained in each mol of human albumin circulating in a human body to achieve the physiological functions of mobilization and storage of fatty acids. A portion of the long-chain fatty acids is lost in the purification process during production of albumin derived from pooled human blood. In accordance with Volume III of the Chinese Pharmacopoeia, 2015 edition, 0.140-0.180 mmol of sodium caprylate shall be added to the pasteurized medical product on the basis of 1 g of albumin; or sodium caprylate and acetyltryptophan are used together with 0.064-0.096 mmol of sodium caprylate and 0.064-0.096 mmol of acetyltryptophan on the basis of 1 g of albumin. Meanwhile, as a large volume injection, the medical product shall meet the physiological conditions of a human body. pH shall be 6.4-7.4, and the osmolality shall be 210-400 mOsmol/kg.

Regardless of which way the recombinant human albumin is expressed, a multi-step purification process including fractionation, chromatography, ultrafiltration and dialysis and the like is required to completely remove proteins, polysaccharides, endotoxins and various contaminants of the host, meanwhile fatty acid ligands are removed completely as well, giving defatted human albumin. Therefore, the stability of recombinant human albumin which has lost the fatty acids or various other ligands from human body is much lower than that of human plasma-derived albumin. Therefore, a quantity of surfactants shall be added at a concentration lower than that of CMC, acting as a hydrophobic patch for the albumin without ligands to prevent aggregation after unfolding due to interactions at various hydrophobic interfaces such as albumin/ air/ container/ oscillation bubbles and the like.

Therefore, based on the General Requirements for Preparations and human serum albumin in the Chinese Pharmacopoeia, American Pharmacopoeia, European Pharmacopoeia and other pharmacopoeias, a certain amount of poloxamer is added to the product and the ratio of long-chain fatty acids is adjusted in the present invention, so that the requirements for human albumin stability are met under the conditions specified in each national pharmacopoeia.

The advantage of the present invention is that by adding long-chain fatty acid ligands directly up to a proper proportion for circulation in a human body together with a small amount of poloxamer, or using existing conventional formulations for human albumin with an additional small amount of poloxamer, human albumin is thermally stable and free from aggregation and fibrillation during storage and shipping. Such aggregates and fibrillar particles are of 50-300 nm in diameter, mainly between 100-200 nm, which can neither be detected by HPLC-SEC test, nor effectively filtered by 0.1-0.2 µm membrane systems, and more importantly, such aggregates are continuously formed in unstable albumin solution systems (even if the aggregates have been completely removed) during storage and shipping, and keep growing into visible particles with a diameter of micron and sub-millimeter level.

The conception, specific structure and resulting technical effects of the present invention will be further described hereinafter in combination with the accompanying drawings to fully understand the purpose, features and effects of the present invention.

### BRIEF DESCRIPTION OF THE DRAWINGS

Fig. 1 is a dynamic light scattering (DLS) chromatogram of sample 1 (human serum albumin containing sodium caprylate and acetyltryptophan) in Embodiment 1.
Fig. 2 is a DLS chromatogram of sample 2 (human serum albumin containing sodium caprylate and acetyltryptophan, with addition of poloxamer) in Embodiment 1.
Fig. 3 is a DLS chromatogram of sample 3 (commercial human serum albumin containing sodium caprylate only) in Embodiment 2.
Fig. 4 is a DLS chromatogram of sample 4 (commercial human serum albumin containing sodium caprylate only, with addition of poloxamer) in Embodiment 2.
Fig. 5 is a DLS chromatogram of sample 5 (recombinant human albumin containing sodium caprylate and acetyltryptophan, without addition of poloxamer or long-chain fatty acids) in Embodiment 3.
Fig. 6 is a DLS chromatogram of sample 6 (recombinant human serum albumin containing sodium caprylate and acetyltryptophan, with addition of long-chain fatty acid (oleic acid) only) in Embodiment 4.
Fig. 7 is a DLS chromatogram of sample 7 (recombinant human albumin containing sodium caprylate and acetyltryptophan, with addition of poloxamer only) in Embodiment 5.
Fig. 8 is a DLS chromatogram of sample 8 (recombinant human albumin containing sodium caprylate and acetyltryptophan, with addition of poloxamer and long-chain fatty acid (oleic acid)) in Embodiment 6.
Fig. 9 is a DLS chromatogram of sample 9 (recombinant human albumin containing sodium caprylate and acetyltryptophan, with addition of poloxamer and long-chain fatty acid (sodium soyate) in Embodiment 7.
Fig. 10 is a DLS chromatogram of sample 10 (recombinant human albumin containing sodium caprylate and acetyltryptophan, with addition of poloxamer and long-chain fatty acids (mixture of sodium stearate and sodium oleate) in Embodiment 8.
Fig. 11 is a DLS chromatogram of sample 11 (recombinant human albumin containing sodium caprylate, with addition of poloxamer and long-chain fatty acids (mixture of sodium stearate and sodium oleate)) in Embodiment 9.

### DETAILED DESCRIPTION OF THE EMBODIMENTS

Embodiments of the present invention are illustrated hereinafter through specific examples. Other advantages and effects of the present invention can be readily understood by those of skill in the art via what is disclosed in the specification. The invention can also be implemented or applied in other different embodiments. The details in this specification can also be modified or changed in various ways based on different views and applications without departing from the spirit of the invention. It shall be noted that the following embodiments and the features in the embodiments can be combined with each other where there is no conflict.

The terms used in this application are defined and explained hereinafter.

The term "human albumin" means human serum albumin or recombinant human albumin, the recombinant human albumin also being called recombinant human serum albumin or recombinant human albumin mutant. Recombinant human albumin comprises at least human albumin derived from microbial genetic recombination, eukaryotic cellular recombinant human albumin, plant transgenic recombinant human albumin and animal transgenic recombinant human albumin and the like.

The term "medical product" shall be understood to comprise at least a solution product, a freeze-dried product or a low-temperature spray-dried product, preferably a solution product which is typically filled aseptically in a glass or plastic container of a defined volume.

The term "human albumin-containing medical product" shall be understood as medical products for clinical injections, culture media, drug excipients, medical devices, vaccine excipients, cosmetics, analytical diagnostic reagents and many other aspects containing human albumin of varied concentrations, for example, a human albumin concentration of 1%-30%, preferably 10%-25%.

Poloxamer is a non-ionic surfactant, a block copolymer of ethylene oxide and 1,2-epoxypropane, with the general formula H(C2H4O) a(C3H6O) b(C2H4O) aOH. With a high degree of biosafety, poloxamer 188 is listed as an emulsifier for intravenous fat emulsions and a stabilizer for antibody injections in the Chinese Pharmacopoeia 2015 edition, the US Pharmacopoeia, the European Pharmacopoeia and in approved drugs

The term "long-chain fatty acids" refers to fatty acids with more than 12 carbon atoms in the carbon chain and should be understood to mean fatty acids of various origins (synthetic or extracted) that are acceptable to humans, or fatty acids present in various forms (e.g., salts, especially sodium salts), including but not limited to one or more from myristic acid (C14:0), palmitic acid (C16:0), stearic acid (C18:0), oleic acid (C18:1), linoleic acid (C18:2), linolenic acid (C18:3) and arachidonic acid (C20:4).

The term "control" with the subject of long-chain fatty acid content in a human albumin-containing medical product, means maintaining the content of long-chain fatty acids in a human albumin-containing medical product within a given range: in a case of long-chain fatty acids inherent in a human albumin-containing medical product prepared by existing methods, such as a human albumin-containing medical product of human blood origin, if the content of long-chain fatty acids is below a given range, "control" means adding long-chain fatty acids during the preparation process of the human albumin-containing medical product such that the amount of long-chain fatty acids in the final human albumin-containing medical product is within a given range; if the content of long-chain fatty acids is already within a given range, "control" means no action is required during the preparation process of the human albumin-containing medical product; if the human albumin-containing medical product prepared by the existing method contains no or a very small amount of long-chain fatty acids, "control" means adding long-chain fatty acids during the preparation process of the human albumin-containing medical product such that the content of long-chain fatty acids in the final human albumin-containing medical product is within a given range.

The term "long-chain fatty acids" shall be understood as a fixed phrase, wherein there is no logical relationship between the "or" and the context of the phrase. For calculation of the content of "long-chain fatty acids", "long-chain fatty acids" refers to the sum of amounts of each long-chain fatty acid. In order to have the content of long-chain fatty acids within a given range, either a long chain fatty acid or a mixture of long-chain fatty acids can be added.

The term "content of long-chain fatty acids" shall be understood as the content of long-chain fatty acids in the final human albumin-containing medical product, comprising the amount of residual long-chain fatty acids during the preparation process due to the long-chain fatty acids inherent in the source materials, and the amount of the additionally added long-chain fatty acids (if any addition during the "controlling" process).

The term "add", when the subject thereof is poloxamer, shall be understood as adding poloxamer during the preparation process of the human albumin-containing medical product such that the poloxamer content in the final human albumin-containing medical product is within a given range.

The steps of "controlling" and "adding" shall be understood as being carried out during the preparation process of the human albumin-containing medical product such that the content of long-chain fatty acids and the content of poloxamer in the final human albumin-containing medical product are each within a given range. For example, the steps can be performed on the final bulk of the formulation, or performed by ultrafiltration and dialysis processes, or poloxamer and long-chain fatty acids can be added as protein stabilizers before pasteurization, or the steps can be performed in the last one or the last few steps of the purification chromatography process, in order to obtain the content range specified in the present invention for poloxamer and long-chain fatty acids in the final human albumin-containing medical product.

The term "improve" shall be understood as a higher stability of the human albumin-containing medical product of the present invention than that of existing human albumin, for example, it is achieved in the present invention that no particle of 30-300 nm in diameter is formed during storage, shipping or ambient-temperature cultivation due to aggregation or fibrillation of human albumin.

The term "particle" shall be understood as a substance of a certain range in diameter and formed during storage, shipping and ambient-temperature cultivation of the human albumin-containing medical product due to aggregation or fibrillation of human albumin, the preferred particle diameter in the present invention being 30-300 nm.

The human albumin-containing medical product of the present invention can be used to inhibit aggregates, agglomerates and fibrillation formed by freeze and thaw in cold drying, freeze drying, and low-temperature spray drying, and to accelerate reconstitution.

The human albumin-containing medical product of the present invention includes but not limited to human serum-derived albumin prepared by Cohn method and polishing, and recombinant human albumin purified by varied steps.

The pH range of the human albumin-containing medical product in the present invention is 6-8, and preferably 6.4-7.4, the pH values in each national pharmacopoeial specification.

The human albumin-containing medical product of the present invention may further comprise diluents, buffers, solubilizers, excipients, pH regulators, sulfur-containing reducing agents, antioxidants and the like. The sulfur-containing reducing agents comprise N-acetyl-DL-tryptophan (N-acetyltryptophan), N-acetyl-L-tryptophan, N-acetylmethionine, N-acetyl-L-methionine, etc. Commonly used buffers and diluents comprise sodium chloride solution, sodium phosphate buffer solution, sodium acetate solution, and sodium citrate solution, preferably within a range according to each national pharmacopoeial specification.

The human albumin-containing medical product of the present invention may be further supplemented with medium-chain fatty acids, such as sodium caprylate, sodium heptanoate, sodium decanoate, and preferably sodium caprylate of the pharmacopoeial specification.

The application of the human albumin-containing medical product of the present invention comprises but not limited to aspects like clinical injections, culture media, drug excipients, medical devices, vaccine excipients, cosmetics and analytical diagnostic reagents.

Embodiments of the present invention are illustrated hereinafter by specific examples.

### Embodiment 1

Commercial human serum albumin (sample 1, Shanghai RAAS blood products Co., Ltd., batch number: 201708A010) was used, the formulation thereof being: human albumin with excipients sodium caprylate and acetyltryptophan. The formulation thereof is in accordance with pharmacopoeial requirements, i.e., 0.079 mmol of sodium caprylate is added on the basis of 1 g of albumin, and 0.076 mmol of acetyltryptophan is added on the basis of 1 g of albumin, and the pH is 6.8. The content of residual long-chain fatty acids after purification of the serum-derived human albumin was tested by gas chromatography. The detailed method of human albumin analysis by gas chromatography (GC) was as follows:

Gas chromatography: model: 7890A; manufacturer: Agilent Technologies Co., Ltd.

GC column: acid-deactivated polyethylene glycol capillary column TG-WAXMS A by Thermo Fisher Scientific Co., Ltd.; specification: 30 m^{∗}0.32 mm^{∗}0.25 µm;

Materials and reagents: C17 heptadecanoic acid: grade: USA reference material; batch number: N-17A-JY16X; strength: >100 mg; content: ≥99.0%; palmitic acid: grade: national certified reference material; batch number: 190032-201603; strength: 200 mg/ampoule; stearic acid: grade: national certified reference material; batch number: 190032-201603; strength: 200 mg/ampoule; oleic acid: grade: national certified reference material; batch number: 111621-2015-06; strength: 100 mg/ampoule; content: ≥99.0%; linoleic acid: grade: USA reference material; batch number: U-59A-J6-Y; strength: >100 mg/ampoule; content: ≥99.0%; linolenic acid: grade: USA reference material; batch number: U-62A-S20-B; strength: >100 mg/ampoule; content: ≥99.0%; chloroform: grade: AR; batch number: 20170410; strength: 500ml/bottle; content: ≥99.0%; manufacturer: Sinopharm.

Experimental method: in accordance with the Pharmacopoeia of the People's Republic of China, 2015 edition: adding the internal standard substance C17 heptadecanoic acid and calculating the absolute peak areas of palmitic acid, stearic acid, oleic acid, linoleic acid, linolenic acid and C17 heptadecanoic acid, plotting a linear regression with the peak area ratio between each fatty acid reference solution and the internal standard against the amount of each fatty acid reference solution, obtaining the linear regression equation, and calculating the absolute peak area of each fatty acid in the test solution, thereby obtaining calculated content of each fatty acid in the test sample.

Chromatography conditions: temperature: 260 °C; column flow: 2.0 ml/min; purge flow: 20 ml/min; split flow: 2 ml/min; temperature program: initial, hold for 5 min at 80°C, increase to 230°C at 10°C/min and hold for 25 min; 45 min in total; detector: model: FID; temperature: 280°C; hydrogen: 40 ml/min; air: 450 ml/min; makeup gas flow rate: 45 ml/min.

The C17 heptadecanoic acid was weighed accurately to prepare the internal standard solution; the fatty acid standards were weighed accurately and mixed as the reference solution. A standard curve was determined to calculate the content of fatty acids in human albumin.

Test results of fatty acid content in human albumin injection (Shanghai RAAS, 201703A010):
Content of C16:0 (palmitic acid) (mol/mol protein): 0.241
Content of C18:0 (stearic acid) (mol/mol protein): 0.147
Content of C18:1 (oleic acid) (mol/mol protein): 0.077
Content of C18:2 (linoleic acid) (mol/mol protein): 0.085
Content of C18:3 (linolenic acid) (mol/mol protein): not detected
Content of C20:4 (arachidonic acid) (mol/mol protein): not detected
Total of the above: proportion of long-chain fatty acids (mol/mol of human albumin): 0.55:1
Dynamic light scattering was employed for testing:
dynamic light scattering (DLS) model: Litesizer 500; manufacturer: Anton Paar (Shanghai) Trading Co., Ltd;
cuvettes: quartz cells;
sample: bulk sample solution was tested.
Method: measurement angle: automatic; temperature range: 25°C-60°C; step: 5°C.

Particles of 30-300 nm due to instability of the commercial human serum albumin needs to be removed by gel filtration; the chromatography conditions thereof are as follows: G-25 packing material (General Electric Company), chromatography device: EV 50D chromatography system (Lisure Science (Suzhou) Co., Ltd.), buffer system: 0.1 mol/L phosphoric acid solution, neutral pH and purified water. The sample was concentrated using a 30K ultrafiltration membrane after chromatography and formulated into a product for testing according to the original product formulation.

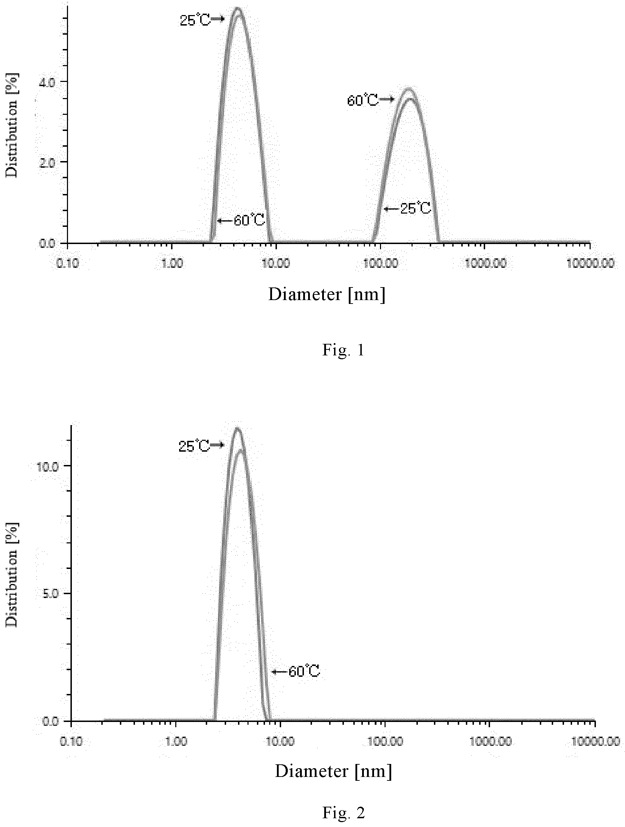

Fig. 1 is a DLS chromatogram of commercial human serum albumin (sample 1).

The particles of 30 nm-300 nm in sample 1 were removed according to the method above, and 75 µg of poloxamer 188 (BASF, batch number: WPAK527B) was added on the basis of 1 g of human albumin to obtain sample 2. The DLS chromatogram of sample 2 is shown in Fig. 2.

As demonstrated by the DLS results in Fig. 1 and Fig. 2, micro-particles were present between 30nm and 300nm in commercial human serum albumin without poloxamer (sample 1) at both 25°C and 60°C, whereas after removal of the particles, micro-particles were absent at both 25°C and 60°C in the human serum albumin with a small amount of additional poloxamer (sample 2).

It was further found by the inventors of the present application through tests that, as a variation of this specific embodiment, poloxamer 188 can also be replaced with one of poloxamer 124, poloxamer 188, poloxamer 237, poloxamer 338 and poloxamer 407, and alternatively, a plurality from poloxamer 188, poloxamer 124, poloxamer 188, poloxamer 237, poloxamer 338 or poloxamer 407 can be used simultaneously. Tests have demonstrated that the same effects could be achieved with these poloxamers, i.e., after removal of particles, micro-particles were absent between 30nm and 300nm at both 25°C and 60°C in human serum albumin with a small amount of additional poloxamer.

The inventors of this application have also found through tests that the desired effect can be achieved by adding only a small amount of poloxamers. In general, with an additional 10 µg or more of poloxamers/gram of human albumin, micro-particles between 30 nm and 300 nm are basically undetectable or only a small amount of micro-particles between 30 nm and 300 nm are present; and with an additional over 500 µg of poloxamers/gram of human albumin, there is a significant increase in micro-particles between 30 nm and 300 nm.

### Embodiment 2

Commercial human serum albumin (sample 3, Shantou Weilun Bio-pharmaceutical Co., Ltd., batch number: 20170104) was used, the formulation ingredients thereof being human albumin with excipients sodium caprylate and sodium chloride. The formulation is in accordance with pharmacopoeial requirements, i.e., 0.159 mmol of sodium caprylate is added on the basis of 1 gram of albumin, with no additional N-acetyltryptophan. The pH is 6.7, and content of total sodium is not more than 160 mmol/L. The method of Embodiment 1 was adopted to determine the content of residual long-chain fatty acids in sample 3, and the proportion of long-chain fatty acids (mol/mol of human albumin) was 0.34:1.

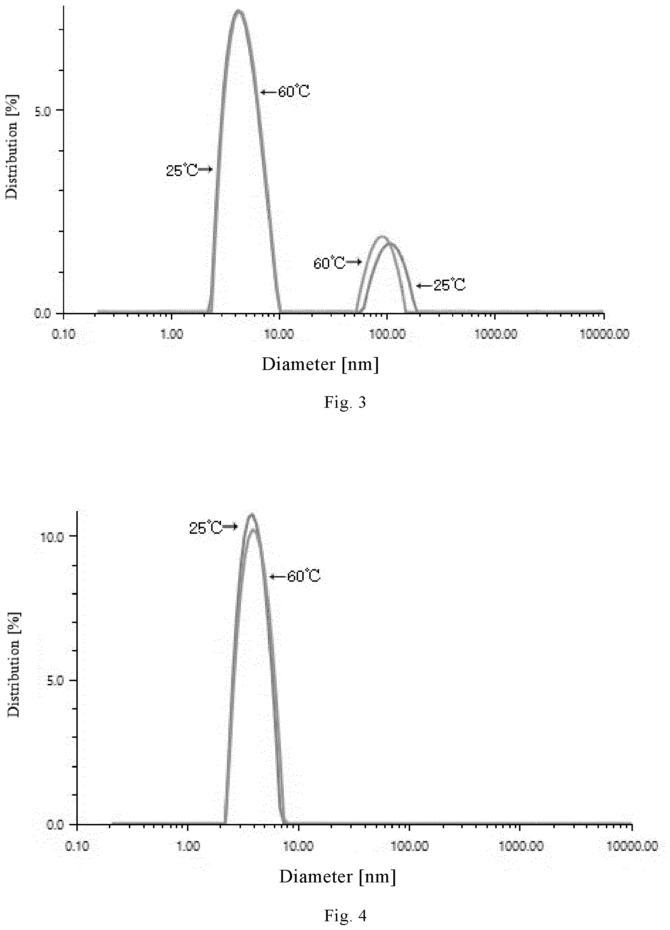

Fig. 3 is a DLS chromatogram of sample 3.

The particles of 30-300 nm in sample 3 were removed according to the method in Embodiment 1, and 100 µg of poloxamer 188 (BASF, batch number WPAK527B) was added on the basis of 1 g of human albumin to obtain sample 4. The DLS chromatogram of sample 4 is shown in Fig. 4.

As shown by the DLS results in Fig. 3 and Fig. 4, micro-particles were present between 30nm and 300nm in the commercial human serum albumin without poloxamer at both 25°C and 60°C. After removal of particles, micro-particles were absent at both 25 °C and 60 °C in the human serum albumin with a small amount of additional poloxamer.

Embodiments 1 and 2 have demonstrated that particles of about 200 nm are present in both standard human serum album formulations of the pharmacopoeia when poloxamer is not added; in a case of a small amount of additional poloxamer, the formation of micro-particles can both be effectively inhibited and prevented.

It was further found by the inventors of the present application through tests that, as a variation of this specific embodiment, poloxamer 188 can also be replaced with one of poloxamer 124, poloxamer 188, poloxamer 237, poloxamer 338 and poloxamer 407, and alternatively, a plurality from poloxamer 188, poloxamer 124, poloxamer 188, poloxamer 237, poloxamer 338 or poloxamer 407 can be used simultaneously. Tests have demonstrated that the same effects could be achieved with these poloxamers, i.e., after removal of particles, micro-particles were absent between 30nm and 300nm at both 25°C and 60°C in human serum albumin with a small amount of additional poloxamer.

The inventors of this application have also found through tests that the desired effect can be achieved by adding only a small amount of poloxamers. In general, with an additional 10 µg or more of poloxamers/gram of human albumin, micro-particles between 30 nm and 300 nm are basically undetectable or only a small amount of micro-particles between 30 nm and 300 nm are present; and with an additional over 500 µg of poloxamers/gram of human albumin, there is a significant increase in micro-particles between 30 nm and 300 nm.

### Embodiment 3

The recombinant human albumin bulk of high purity obtained after improved purification according to the method in the Chinese patent application (application no. 201010124935.2) was used herein. The tested residual content of long-chain fatty acids of the purified recombinant human albumin as indicated by the proportion of long-chain fatty acids (mol/mol of human albumin) equaled to 0.059:1, which is nearly 10 times lower than the content of long-chain fatty acids in human serum-derived albumin. Without any addition of long-chain fatty acids or poloxamers, sample 5 was prepared according to the method requirements of the Chinese Pharmacopoeia 2015 edition only, i.e., 0.071 mmol of sodium caprylate (Chengdu Huayi Pharmaceutical Excipients Manufacturing Co., Ltd, batch No. 20170901) was added on the basis of 1 g of albumin, and 0.085 mmol of acetyltryptophan (Adamas Reagent Co., Ltd, batch No. P230264) was added on the basis of 1 g of albumin; pH was 6.8; and the total sodium content was controlled to be not more than 160 mmol/L.

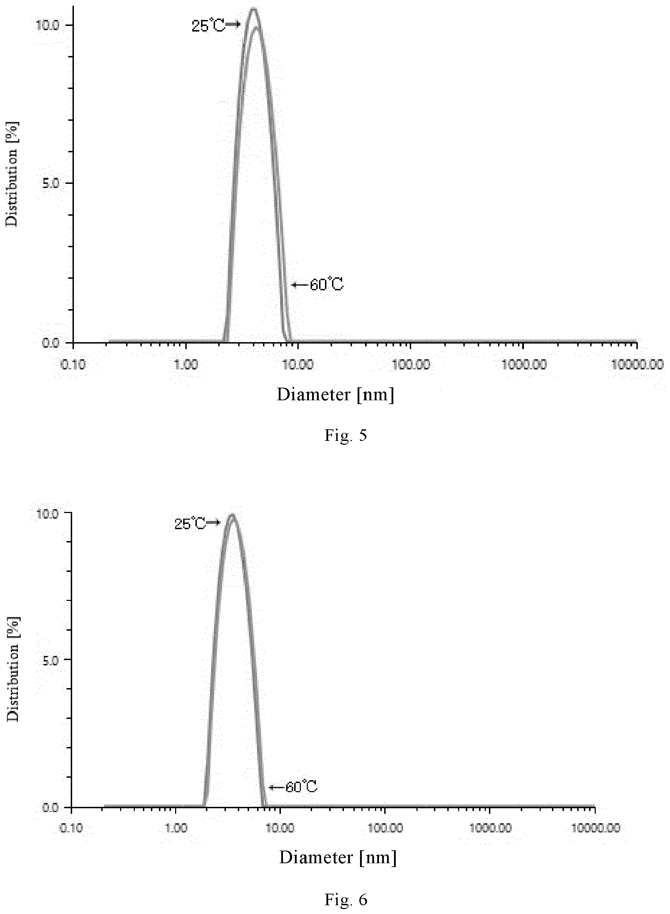

Fig. 5 is a DLS chromatogram of the recombinant human albumin without addition of poloxamer or long-chain fatty acids (sample 5).

### Embodiment 4

Preparation of sample 6: taking the recombinant human albumin bulk of high purity obtained by the method of Embodiment 3 above, adding sodium oleate (BBI Life Sciences, batch number: E802BA0026) to the bulk till an 1.0:1.0 molar ratio between long-chain fatty acids and recombinant human albumin is achieved (residual long-chain fatty acids from the bulk were counted), adding 0.081 mmol of sodium caprylate (Chengdu Huayi Pharmaceutical Excipients Manufacturing Co., Ltd, batch No. 20170901) on the basis of 1 g of albumin, and adding 0.067mmol acetyltryptophan (Adamas Reagent Co., Ltd Batch No. P230264) on the basis of 1 g of albumin, pH being 6.7, and controlling the total sodium to be not more than 160 mmol/L.

Fig. 6 is a DLS chromatogram of the recombinant human albumin-containing medical product with addition of long-chain fatty acids only (sample 6).

### Embodiment 5

Preparation of sample 7: taking the recombinant human albumin bulk of high purity obtained by the method of Embodiment 3 above, adding 100 µg of poloxamer 188 (BASF, batch no. WPAK527B) on the basis of 1 g of human albumin, adding 0.079 mmol of sodium caprylate (Chengdu Huayi Pharmaceutical Excipients Manufacturing Co., Ltd, batch No. 20170901) on the basis of 1 g of albumin, and adding 0.066mmol of acetyltryptophan (Adamas Reagent Co., Ltd Batch No. P230264) on the basis of 1 g of albumin, pH being 6.6, and controlling the total sodium to be not more than 160 mmol/L.

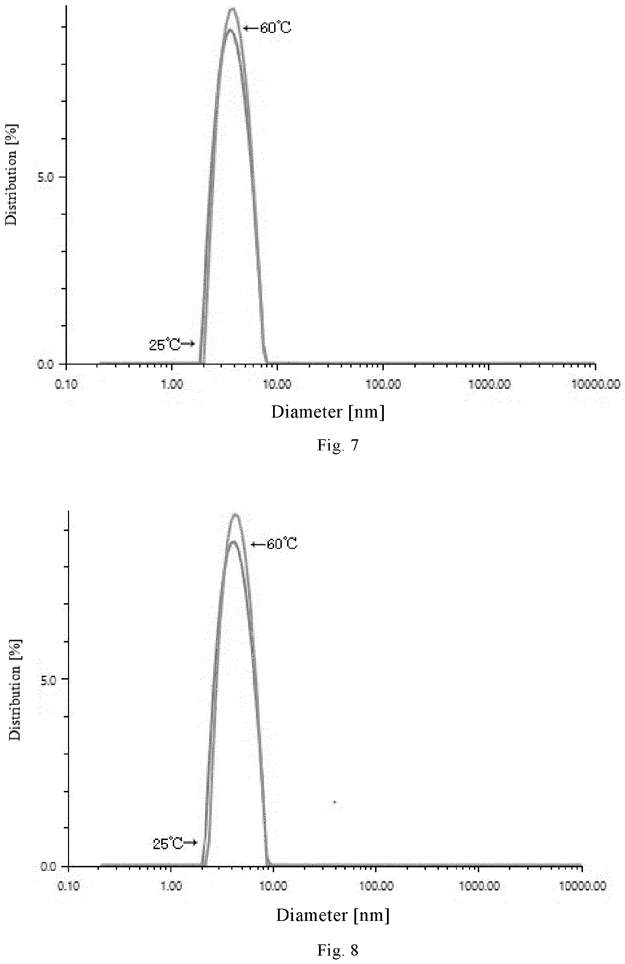

Fig. 7 is a DLS chromatogram of the recombinant human albumin with addition of poloxamer only (sample 7).

### Embodiment 6

Preparation of sample 8: taking the recombinant human albumin bulk of high purity obtained by the method of the Embodiment 3 above, adding 100 µg of poloxamer 188 (BASF, batch no. WPAK527B) on the basis of 1 gram of human albumin, adding sodium oleate (BBI Life Sciences, batch number: E802BA0026) till an 1.0:1.0 molar ratio between long-chain fatty acids and recombinant human albumin was achieved (residual long-chain fatty acids from the bulk were counted), adding 0.083 mmol of sodium caprylate (Chengdu Huayi Pharmaceutical Excipients Manufacturing Co., Ltd, batch No. 20170901) on the basis of 1 g of albumin, and adding 0.084mmol of acetyltryptophan (Adamas Reagent Co., Ltd Batch No. P230264) on the basis of 1 g of albumin, pH being 6.8, and controlling the total sodium to be not more than 160 mmol/L.

Fig. 8 is a DLS chromatogram of the recombinant human albumin with addition of poloxamer and long-chain fatty acids (sample 8).

### Embodiment 7

Preparation of sample 9: taking the recombinant human albumin bulk of high purity obtained by the method of Embodiment 3 above, adding 100 µg of poloxamer 188 (BASF, batch no. WPAK527B) on the basis of 1 g of human albumin, adding sodium soyate (Qingdao Raynol Chemical Co., Ltd., batch no. 20190121) till a 0.5:1.0 molar ratio between long-chain fatty acids and recombinant human albumin was achieved (residual long-chain fatty acids from the bulk were counted), adding 0.082 mmol of sodium caprylate (Chengdu Huayi Pharmaceutical Excipients Manufacturing Co., Ltd, batch No. 20170901) on the basis of 1 g of albumin, and adding 0.080mmol of acetyltryptophan (Adamas Reagent Co., Ltd, batch No. P230264) on the basis of 1 g of albumin, pH being 6.9, and controlling the total sodium to be not more than 160 mmol/L.

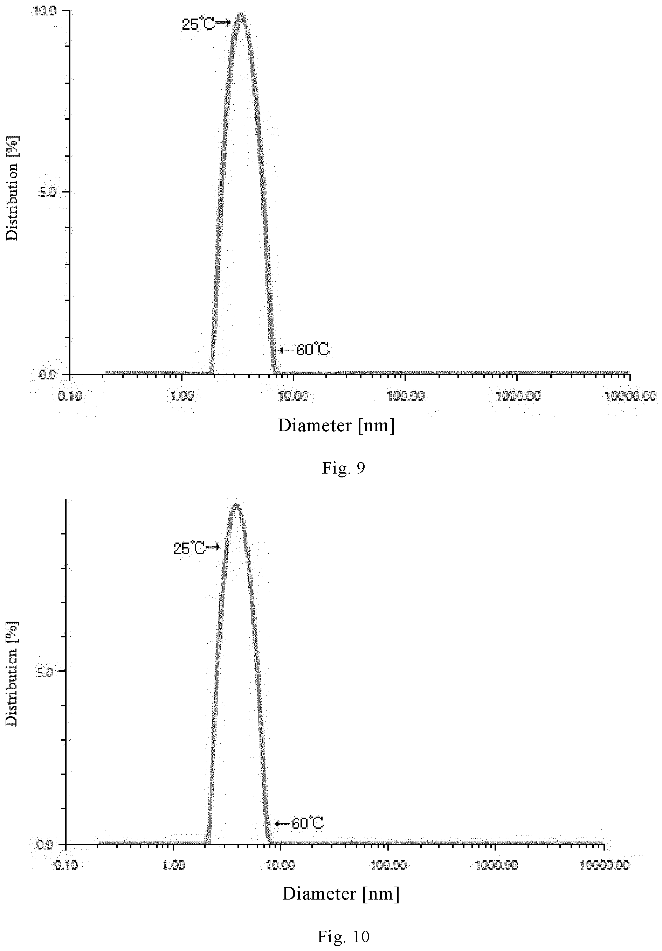

Fig. 9 is a DLS chromatogram of the recombinant human albumin with addition of poloxamer and long-chain fatty acid (sodium soyate) (sample 9).

### Embodiment 8

Preparation of sample 10: taking the recombinant human albumin bulk of high purity obtained by the method of Embodiment 3 above, adding 50 µg of poloxamer 188 (BASF, batch no. WPAK527B) on the basis of 1 g of human albumin, mixing well sodium stearate (Damas-beta, batch number: P1042477) and sodium oleate (BBI Life Sciences, batch number: E802BA0026) in a molar ratio of 1:2 and adding to the bulk till a 0.6:1.0 molar ratio between mixed long-chain fatty acids and recombinant human albumin was achieved (residual long-chain fatty acids from the bulk were counted), adding 0.086 mmol of sodium caprylate (Chengdu Huayi Pharmaceutical Excipients Manufacturing Co., Ltd, batch No. 20170901) on the basis of 1 g of albumin, and adding 0.076 mmol of acetyltryptophan (Adamas Reagent Co., Ltd Batch No. P230264) on the basis of 1 g of albumin, pH being 7.0, and controlling the total sodium to be not more than 160 mmol/L.

Fig. 10 is a DLS chromatogram of the recombinant human albumin with additional poloxamer and long-chain fatty acids (mix of sodium stearate and sodium oleate) (sample 10).

### Embodiment 9

Preparation of sample 11: taking the recombinant human albumin bulk of high purity obtained by the method of Embodiment 3 above, adding 50 µg of poloxamer 188 (BASF, batch no. WPAK527B) on the basis of 1 g of human albumin, mixing well sodium stearate (Damas-beta, batch number: P1042477) and sodium oleate (BBI Life Sciences, batch number: E802BA0026) in a molar ratio of 1:2 and adding to the bulk till a 0.6:1.0 molar ratio of long-chain fatty acid mixture to recombinant human albumin was achieved (residual long-chain fatty acids from the bulk were counted), adding 0.153 mmol of sodium caprylate (Chengdu Huayi Pharmaceutical Excipients Manufacturing Co., Ltd, batch No. 20170901) on the basis of 1 g of albumin, pH being 7.0, and controlling the total sodium to be not more than 160 mmol/L.

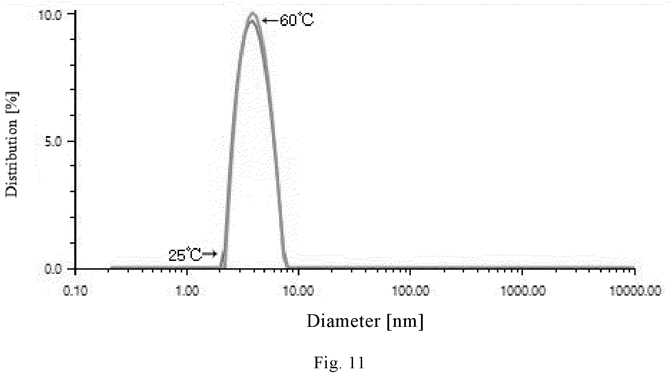

Fig. 11 is a DLS chromatogram of the recombinant human albumin with addition of poloxamer and long-chain fatty acid (sodium stearate/ sodium oleate mixture) (sample 11).

### Embodiment 10

Incubation and thermostability tests of the samples prepared in Embodiments 1-9 (Samples 1-11). The method thereof was in accordance with the Chinese Pharmacopoeia, 2015 edition, Volume III, p. 244-245. The results are shown in Table 1.

**Table 1 Observation results for stability of samples 1-11**

| Sample No. | Incubation at 57°C for 50 hours | Incubation at 30°C for 14 days | Incubation at 20°C for 28 days |
|---|---|---|---|
| Sample 1 | (-) | (-) | (-) |
| Sample 2 | (-) | (-) | (-) |
| Sample 3 | (-) | (-) | (-) |
| Sample 4 | (-) | (-) | (-) |
| Sample 5 | (+) | (+) | (+) |
| Sample 6 | (+) | (+) | (+) |
| Sample 7 | (+) | (+) | (+) |
| Sample 8 | (-) | (-) | (-) |
| Sample 9 | (-) | (-) | (-) |
| Sample 10 | (-) | (-) | (-) |
| Sample 11 | (-) | (-) | (-) |

| | | | |
|---|---|---|---|
| (Note: - is negative indicating qualified; + is positive indicating unqualified) | | | |

The above embodiments demonstrated the effect of oleic acid, sodium soyate, mixture of sodium stearate and sodium oleate, and mixture of sodium stearate and sodium oleate, in combination with poloxamer in promoting the stability of recombinant human albumin products. It was further found by the inventors of the present application through tests that, as a variation of the embodiments, the long-chain fatty acids can also be one or more selected from myristic acid (C14:0), palmitic acid (C16:0), stearic acid (C18:0), oleic acid (C18:1), linoleic acid (C18:2), linolenic acid (C18:3) and arachidonic acid (C20:4). Tests have demonstrated that the same effect can be achieved by these long-chain fatty acids, i.e., the combined use thereof with poloxamers can promote the stability of recombinant human albumin products. Negative results were shown in the above stability test.

It was further found by the inventors of the present application through tests that, as a variation of the above embodiments, poloxamer 188 can also be replaced with one of poloxamer 124, poloxamer 188, poloxamer 237, poloxamer 338 and poloxamer 407, and alternatively, a plurality from poloxamer 188, poloxamer 124, poloxamer 188, poloxamer 237, poloxamer 338 or poloxamer 407 can be used simultaneously. Tests have demonstrated that the same effect can be achieved by these poloxamers; the stability of human serum albumin can meet requirements with the addition of a quantity of long-chain fatty acids and a small amount of poloxamer.

The inventors of this application have also found through tests that the desired effect can be achieved by adding only a small amount of poloxamer. In general, the observation results for stability are substantially negative when 10 µg or more of poloxamer/g of human albumin is added; and when more than 500 µg of poloxamer/g of human albumin is added, positive results start to appear in the observation tests for stability.

It was further found by the inventors of the present application through tests that, the stability of human albumin medical product is at its best when the molar ratio between long-chain fatty acids and human albumin is 0.2-3.0, and particularly 0.3-2.0. When the molar ratio between long-chain fatty acids and human albumin is lower than 0.3 or greater than 2.0, positive results start to appear in a small amount in the observation tests for stability, whereas when the molar ratio between long-chain fatty acids and human albumin is lower than 0.2 or greater than 3.0, positive results start to appear in a large amount in the observation tests for stability

### Embodiment 11

Samples 5 to 11 prepared by the method of Embodiments 3 to 9 were subjected to differential scanning calorimetry (DSC) analysis and DSC chromatograms were obtained. DSC model: Nano DSC 602000; manufacturer: TA; sample diluent: 0.01 mol/L phosphoric acid with a neutral pH. Test conditions: the bulk sample was diluted with the diluent to 1-5 mg/ml; temperature range: 45°C-90°C; step: 1°C/min. The results are shown in Table 2.

**Table 2 DSC results of samples 5-11**

| Sample no. | Tₒₙₛₑₜ | Tₘ₁ | Tₘ₂ |
|---|---|---|---|
| Recombinant human albumin bulk of high purity | 60.9°C | 65.0°C | 70.1°C |
| Sample 5 | 62.9°C | 66.7°C | 70.5°C |
| Sample 6 | 63.2°C | 68.0°C | 73.7°C |
| Sample 7 | 62.8°C | 67.0°C | 71.6°C |
| Sample 8 | 63.3°C | 68.3°C | 74.2°C |
| Sample 9 | 64.0°C | 68.7°C | 72.9°C |
| Sample 10 | 63.6°C | 68.2°C | 70.0°C |
| Sample 11 | 64.7°C | 68.8°C | 73.1°C |

The results above of the present invention have demonstrated a synergistic stabilizing effect of poloxamers and long-chain fatty acids. For human blood-derived albumin preparations, the inherent residual long-chain fatty acids and other ligands from circulation in human body can effected inhibit the appearance of particles of 30-300 nm and improve thermostability with only a small amount of additional poloxamers while no further addition of long-chain fatty acids is required.

For recombinant human albumin of high purity, which is substantially defatted completely, additional poloxamers are required to inhibit the appearance of particles of 30-300 nm. Comparison between the test results demonstrates that, in a case where only poloxamers, conventional pharmacopoeial sodium caprylate (medium-chain fatty acids) and acetyltryptophan are added, requirements for thermostability can not be met due to a lack of mutual support for the various adjacent domain ligands of albumin by long-chain fatty acids, although medium-chain fatty acids can effectively enhance Tonset, Tm1 and Tm2.

Therefore, controlling the content of long-chain fatty acids can effectively enhance Tonset, Tm1 and Tm2, and while the connective stability of the protein domains are enhanced, a small amount of additional poloxamers is required to inhibit hydrophobic interactions. The human albumin-containing medical product of the present invention meets the requirements of stability for incubation at 57°C for 50 hours, at 30°C for 14 days and at 20°C for 28 days with excellent stability indicators, meeting requirements of stability for long-term storage and shipping at 2°C-8°C and room temperature.

The embodiments above are merely illustrative of the principles of the invention and efficacy thereof, and are not intended to limit the invention. Any person of the skill in the art may modify or change the above embodiments given that it is not against the spirit and scope of the present invention. Therefore, all equivalent modifications or changes made by those with ordinary knowledge in the art, without departing from the spirit and technical concept revealed by the present invention, shall still be covered by the claims of the present invention.

## Claims

1. A preparation method of a human albumin-containing medical product, wherein the method comprises: improving the stability of human albumin in the medical product by controlling the content of long-chain fatty acids in the medical product and adding poloxamer to the medical product.

2. The method according to claim 1, wherein the method further comprises: improving the stability of human albumin in the medical product by reducing aggregation and fibrillation of human albumin.

3. The method according to claim 1, wherein the method further comprises: improving the stability of human albumin in the medical product so that no particle of over 30 nm in diameter is formed from aggregation and/or fibrillation of human albumin after the preparation of the medical product.

4. The method according to claim 1, wherein the method further comprises: improving the stability of human albumin in the medical product so that no particle of over 100 nm in diameter are formed from aggregation and/or fibrillation of human albumin after the preparation of the medical product.

5. The method according to claim 1, wherein the method further comprises: improving the stability of human albumin in the medical product so that no particle of about 200 nm in diameter is formed from aggregation and/or fibrillation of human albumin after the preparation of the medical product.

6. The method according to claim 1, wherein the method further comprises: improving the stability of human albumin in the medical product so that the stability meets requirements in local pharmacopoeias.

7. The method according to claim 1, wherein the method further comprises: improving the stability of human albumin in the medical product so that the stability meets the following conditions: no turbidity or visible particles are formed during incubation at 57°C for 50 hours, at 30°C for 14 days and at 20°C for 28 days.

8. The method according to claim 1, wherein the human albumin is human serum albumin, and the method comprises: improving the stability of human albumin in the medical product by adding poloxamer.

9. The method according to claim 1, wherein the human albumin is recombinant human albumin, and the method comprises: improving the stability of human albumin in the medical product by adding long-chain fatty acids and poloxamer.

10. The method according to claim 9, wherein the long-chain fatty acids are added in the form of salts.

11. The method according to claim 9, wherein the long-chain fatty acids are added in the form of sodium salts.

12. The method according to claim 1, wherein the method further comprises: after the preparation of the medical product, the molar ratio of the long-chain fatty acids and human albumin is 0.2-3.0.

13. The method according to claim 1, wherein the method further comprises: after the preparation of the medical product, the molar ratio of the long-chain fatty acids and human albumin is 0.3-2.0.

14. The method according to claim 1, wherein the method further comprises: after the preparation of the medical product, the content of the poloxamer is 10-500 µg/ g of human albumin.

15. The method according to claim 1, wherein the poloxamer is one or more selected from poloxamer 124, poloxamer 188, poloxamer 237, poloxamer 338 and poloxamer 407.

16. The method according to claim 1, wherein the poloxamer is poloxamer 188.

17. The method according to claim 1, wherein the long-chain fatty acid is one or more selected from myristic acid (C14:0), palmitic acid (C16:0), stearic acid (C18:0), oleic acid (C18:1), linoleic acid (C18:2), linolenic acid (C18:3) and arachidonic acid (C20:4).

18. The method according to claim 1, wherein the long-chain fatty acid is soya fatty acid or fully hydrogenated soya fatty acid.

19. The method according to claim 1, wherein the method further comprises: adding ingredients and contents as required by local pharmacopoeias.

20. The method according to claim 19, wherein the ingredients required by local pharmacopoeias comprise sodium caprylate or/and N-acetyltryptophan.

21. A human albumin-containing medical product prepared according to the method of claim 1.

22. A human albumin-containing medical product, wherein the medical product contains no particle of over 30 nm in diameter due to aggregation and/or fibrillation of human albumin.

23. A human albumin-containing medical product, wherein the medical product contains no particle of over 100 nm in diameter due to aggregation and/or fibrillation of human albumin.

24. A human albumin-containing medical product, wherein the medical product contains no particle of about 200 nm in diameter due to aggregation and/or fibrillation of human albumin.

25. A human albumin-containing medical product, wherein the method further comprises: the stability of human albumin in the medical product meets the following conditions: no turbidity or visible particles are formed during incubation at 57°C for 50 hours, at 30°C for 14 days and at 20°C for 28 days.

26. A human albumin-containing medical product, wherein the medical product comprises poloxamer and long-chain fatty acids.

27. The medical product according to claim 26, wherein the molar ratio of the long-chain fatty acids and human albumin in the medical product is 0.2-3.0.

28. The medical product according to claim 26, wherein the molar ratio of the long-chain fatty acids and human albumin in the medical product is 0.3-2.0.

29. The medical product according to claim 26, wherein the method further comprises: the content of poloxamer in the medical product is 10-500 µg/ g of human albumin.

30. The medical product according to claim 26, wherein the poloxamer is one or more selected from poloxamer 124, poloxamer 188, poloxamer 237, poloxamer 338 and poloxamer 407.

31. The medical product according to claim 26, wherein the poloxamer is poloxamer 188.

32. The medical product according to claim 26, wherein the long-chain fatty acid is one or more selected from myristic acid (C14:0), palmitic acid (C16:0), stearic acid (C18:0), oleic acid (C18:1), linoleic acid (C18:2), linolenic acid (C18:3) and arachidonic acid (C20:4).

33. The medical product according to claim 26, wherein the long-chain fatty acid is soya fatty acid or fully hydrogenated soya fatty acid.

34. The medical product according to claim 26, wherein the medical product further comprises ingredients and contents required by local pharmacopoeias.

35. The medical product according to claim 34, wherein the ingredients required by local pharmacopoeias comprise sodium caprylate or/and N-acetyltryptophan.
